# EUROPÄISCHE PATENTSCHRIFT

(11) **EP 0 951 873 B1**
(45) Veröffentlichungstag und Bekanntmachung des Hinweises auf die Patenterteilung: **02.07.2003**
(21) Anmeldenummer: 99250114.8
(22) Anmeldetag: 13.04.1999
(51) Int. Cl.: A61B 17/17

(54) **Zielgerät zur röntgenfreien proximalen und distalen Verriegelung von Marknägeln**
Aiming device for a X-ray free proximal and distal locking of an intramedullary nail
Dispositif de visée pour le verrouillage proximal et distal d'un clou intramédullaire

(30) Priorität: 24.04.1998 DE 19819168
(43) Veröffentlichungstag der Anmeldung: 27.10.1999
(73) Patentinhaber: aap Implantate AG, 12099 Berlin (DE)
(72) Erfinder: Abel, Christian, Dipl.-Ing., 10623 Berlin (DE)
(74) Vertreter: Meissner, Peter E., Dipl.-Ing.

(56) Entgegenhaltungen:
- DE-U- 29 608 071
- FR-A- 2 559 376

## Beschreibung

Die Erfindung betrifft ein Zielgerät zur röntgenfreien proximalen und distalen Verriegelung von Marknägeln, nach dem Oberbegriff des Anspruchs 1.

Um für den Operateur zusätzliche Strahlenbelastung zu vermeiden, ist es bekannt, eine Zielvorrichtung für das Setzen der Verriegelungsschrauben für den im Knochen eingebrachten Marknagel starr mit diesem zu verbinden. Die Zielvorrichtung weist Führungsbohrungen auf, durch die sowohl die Knochenbohrung als auch die Verriegelungsschraube genau fluchtend mit den Konturen der Bohrung bzw. Rinne am Nagel eingebracht werden kann.

Während zunächst nur das proximale Zielgerät fest mit dem Marknagel verbunden war und ein separates Zielgerät für das Setzen der distalen Verriegelungsschrauben mit Hilfe von Betrachtung unter Röntgenstrahlung ausgerichtet werden mußte, weist der Stand der Technik heute bereits Zielgeräte aus, bei denen eine zusätzliche feste Verbindung zwischen proximaler und distaler Zielvorrichtung besteht.

So ist nach der DE 42 40 277 A1 eine Hilfsschiene mit Führungslöchern für einen anzusetzenden Knochenbohrer vorgesehen, die am proximalen Ende des Marknagels angeschraubt ist und in die in einem bestimmten Abstand, analog zum Marknagel, Führungslöcher eingebracht sind.

Die Hilfsschiene ist im Bereich der distalen Führungslöcher mit Hilfe von Befestigungsstiften, z.B. Kirschnerdraht, auch unter Zuhilfenahme eines zusätzlichen Brückenbügels, hinsichtlich seiner Lage zum Knochen festgelegt.

Die DE 296 08 071 U1, auf die der Oberbegriff des ersten Anspruchs basiert ist, geht hinsichtlich der Festlegung der distalen Zielvorrichtung noch einen Schritt weiter, indem hier zusätzlich zu einer longitudinalen Zielschiene, mit der die distale Zielvorrichtung in einem bestimmten Abstand zur proximalen Zielvorrichtung gehalten wird, noch ein Abstandsgeber vorgesehen ist, mittels dessen die Ausrichtung der Bohrungen in einer an der Zielschiene befestigten Bohrlehre zu den Boh-rungen im Marknagel erfolgt. Von der Anmelderin wird eine Zielanordnung gefertigt, bei der die proximale und die distale Zielvorrichtung jeweils aus einem U-förmigen Teil besteht, wobei in den verbreiterten Endbereichen der Schenkel der U-förmigen Teile Führungsbohrungen eingebracht sind, der Marknagel über einen Steg fest mit der proximalen Zielvorrichtung verbunden ist und zwischen der proximalen und distalen Zielvorrichtung eine verstellbare Abstandshalterung vorgesehen ist. Die übereinstimmende Lage der Führungsbohrungen der distalen Zielvorrichtung zu den die Verriegelungsschrauben aufnehmenden Rinnen im Marknagel wird durch ein verstell- und arretierbares Abstandselement sichergestellt, das, durch eine Bohrung im Knochen geführt, in der endgültigen Position am Marknagel anliegen muß.

Während nach DE 42 40 277 A1 das Fluchten der distalen Bohrungen in der Hilfsschiene und im Marknagel durch das vorherige Setzen eines dünneren Befestigungsstiftes durch eine der Bohrungen erreicht werden soll, woran sich eine Fixierung mittels weiterer im Knochen verankerter Befestigungsstifte anschließt, gibt die Anordnung eines am distalen Zielgerät befestigten Abstandselementes, das am Marknagel zur Anlage gebracht wird, bereits wesentlich mehr Sicherheit für die Einhaltung der Fluchtung der kommunizierenden Bohrungen.

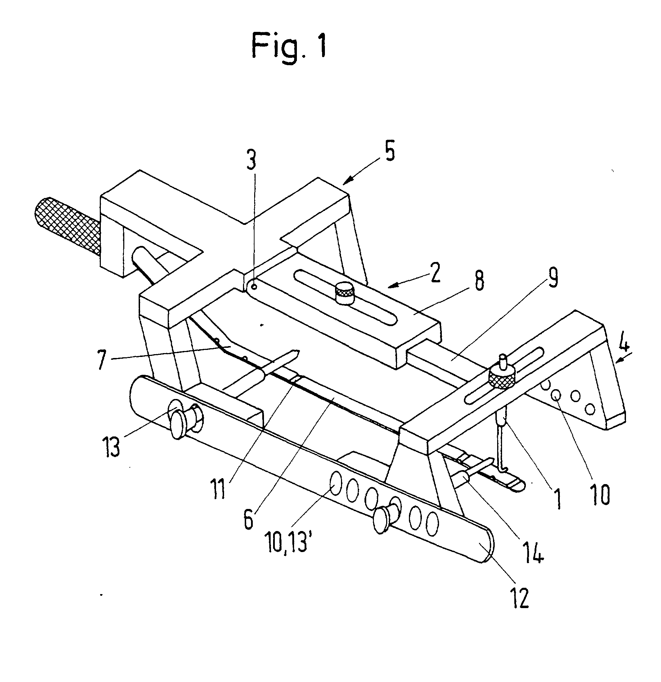

Bei der letztgenannten Lösung tritt jedoch folgendes Problem auf: Wenn sich der Marknagel beim Einsetzen in den unaufgebohrten Knochen in anteriorer oder posteriorer Richtung verbiegt, wird die distale Zielvorrichtung 4 über das Abstandselement 1 (Fig.1) mit angehoben und schwenkt über den Abstandshalter 2 um das Gelenk 3 an der proximalen Zielvorrichtung 5.

Geht man davon aus, daß der Drehpunkt des Nagels 6 im wesentlichen im Bereich der Herzogkrümmung 7 liegt, dann ergeben sich für den Nagel 6 und den Abstandhalter 2 jeweils in einer anderen Ebene liegende Drehpunkte, wobei die Ebenen um die Länge des Abstandselements 1 voneinander entfernt sind.

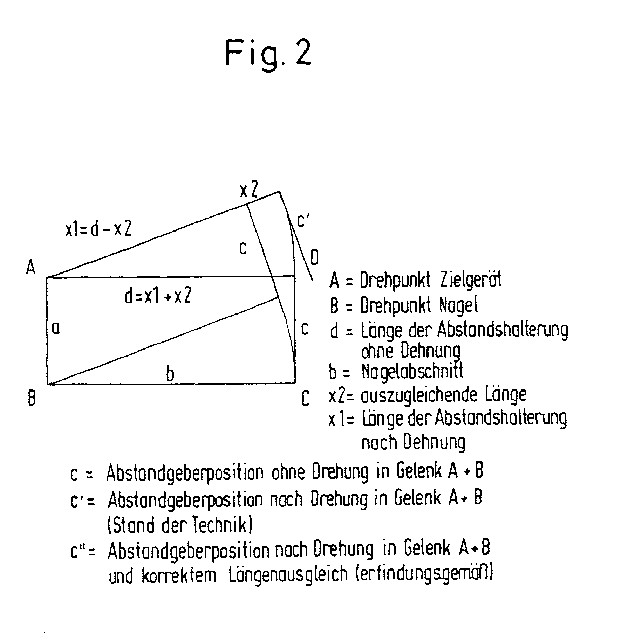

Aus der Fig. 2 ist erkennbar, daß sich die Mittelachsen der Bohrungen in dem distalen Zielgerät bei Auslenkung um den Winkel α um den Betrag x2 auf einen weiter am Ende des Nagels liegenden Bereich von den Mittenachsen der ohne Auslenkung kommunizierenden Rinnen im Marknagel entfernen. Das führt dazu, daß bei einem gebogenen (geschwenkten) Nagel die Rinnen von den eingelegten Verriegelungsschrauben nicht mehr getroffen werden können.

Der Erfindung lag die Aufgabe zugrunde, die Zielvorrichtung so zu gestalten, daß auch bei gekrümmtem (geschwenktem) Nagel die distale Zielvorrichtung derart mitschwenkt, daß die Fluchtung der kommunizierenden Bohrungen in der Zielvorrichtung bzw. Kerben im Marknagel erhalten bleibt.

Die Aufgabe wird entsprechend den Merkmalen des Anspruchs 1 gelöst.

Die Unteransprüche geben zweckmäßige Ausgestaltungen des Anspruchs 1 wieder.

Die Erfindung soll an einem Ausführungsbeispiel erläutert werden.

In den Zeichnungen zeigen:
- Fig. 1:: Zielgerät entsprechend der Erfindung
- Fig. 2:: schematische Darstellung der Längen- und Lageverhältnisse bei ungekrümmtem und bei gekrümmtem (geschwenktem) Marknagel.

Durch das Verbindungselement 12 wird der Drehpunkt für die distale Zielvorrichtung 4 in die Ebene der Mittenachsen der Führungsbohrungen 10 bzw. der Marknagelkerben 11 gelegt. Ohne diese wesentliche erfindungsgemäße Maßnahme kann es zu folgendem Ablauf kommen:
Bei einem durch das Einbringen in den Knochen bedingten Schwenken des Marknagels 6 im Bereich der Herzogkrümmung 7 wird die distale Zielvorrichtung 4 über das Abstandselement 1 angehoben. Wie aus Fig. 2 hervorgeht, wandert in Abhängigkeit vom Drehwinkel α die senkrechte Projektion des Endes der Abstandshaltung 2 und damit die Projektion der Führungsbohrungen 10 in der distalen Zielvorrichtung 4 weiter zum Ende des Marknagels 6 und eine Fluchtung der Mittenachsen der Führungsbohrungen 10 in der distalen Zielvorrichtung 4 und der Mittenachsen der korrespondierenden Rinnen 11 im Marknagel ist nicht mehr gegeben.

Bei der erfindungsgemäßen Lösung (Fig.1) schwenkt die distale Zielvorrichtung 4 durch das drehbar an den Schenkeln des U-förmigen Teiles der proximalen und distalen Zielvorrichtung gelagerte Verbindungselement 12 so wie der Marknagel 6 in der gleichen Ebene, d. h. die Fluchtung der Mittenachsen wird aufrecht erhalten. Bei dieser Schwenkung verkürzt sich die Abstandshalterung 2 durch teleskopartiges Einfahren des Teiles 9 der Abstandshalterung in das Teil 8.

Das erfindungsgemäße Verbindungselement 12 ist als Flachstahlstreifen ausgebildet, welcher Bohrungen 13,13' am proximalen und distalen Ende aufweist.

Das Verbindungselement 12 ist am Schenkel des U-förmigen Teiles der proximalen bzw. distalen Zielvorrichtung (4, 5) beispielsweise dadurch drehbar gelagert, daß jeweils eine Gewebeschutzhülse 14, die bei der Operation in die Führungsbohrungen 10 eingesetzt wird, zusätzlich durch die Bohrungen 13, 13' gesteckt ist.

Zur Berücksichtigung der ventralen oder dorsalen Lage der Marknagelrinnrn 11 sind die Führungsbohrungen im Schenkel des U-förmigen Teils der Zielvorrichtungen 4, 5 in zwei Ebenen angeordnet. Der jeweilige Abstand zwischen proximaler und distaler Zielvorrichtung 4, 5 ist beispielsweise in 10 mm-Schritten dadurch einstellbar, daß am proximalen Ende des Verbindungselements 12 zwei Bohrungen 13 im 10 mm-Abstand und am distalen Ende des Verbindungselements 12 eine Vielzahl von Bohrungen 13' im 20 mm-Abstand eingebracht sind.

## Patentansprüche

1. Zielgerät zur röntgenfreien proximalen und distalen Verriegelung von Marknägeln, bei dem die proximale (5) und die distale Zielvorrichtung (4) jeweils aus einem U-förmigen Teil besteht, in deren Schenkel in den verbreiterten Endbereichen Führungsbohrungen (10) eingebracht sind und bei dem der Marknagel (6) über einen Steg fest mit der proximalen Zielvorrichtung (5) und die proximale Zielvorrichtung mit der distalen Zielvorrichtung (4) über eine schwenk- und verstellbare Abstandshalterung (2) verbunden ist und bei dem der Abstand des Marknagelendes von der distalen Zielvorrichtung durch ein Abstandselement (1) derart eingestellt ist, daß die Mittenachsen der Führungsbohrungen (10) in den Zielvorrichtungen mit den Mittenachsen von mit den Führungsbohrungen (10) kommunizierenden Rinnen (11) oder Bohrungen im Marknagel (6) fluchten,
**dadurch gekennzeichnet,**
**daß** die Abstandshalterung (2) längenveränderlich aus zwei teleskopartig ineinanderschiebbaren Teilen (8, 9) besteht, daß der Schenkel der U-förmigen proximalen Zielvorrichtung (5) mit dem Schenkel der U-förmigen distalen Zielvorrichtung (4) in der Ebene der Mittenachsen der Führungsbohrungen (10) bzw. der Marknagelrinnen (11) durch ein starres Verbindungselement (12), das jeweils drehbar an dem Schenkel der U-förmigen proximalen bzw. distalen Zielvorrichtung (4, 5) gelagert ist, verbunden ist und daß die Abstandshalterung (2) über ein Gelenk (3) senkrecht zur Ebene der Mittenachsen der Führungsbohrungen (10) bzw. Marknagelrinnen (11) schwenkbar an der proximalen Zielvorrichtung (5) angeordnet ist.

2. Zielgerät nach Anspruch 1,
**dadurch gekennzeichnet,**
**daß** das Verbindungselement (12) ein Flachstahlstreifen ist, in den entsprechend dem Abstand der Führungsbohrungen (10) in der proximalen bzw. distalen Zielvorrichtung (4, 5) Bohrungen (13, 13') eingebracht sind, und eine bei der Operation bekanntermaßen verwendete Gewebeschutzhülse (14) durch die Bohrungen (13,13') und die Führungsbohrungen (10) gesteckt ist.

3. Zielgerät nach Anspruch 2,
**dadurch gekennzeichnet,**
**daß** zur Erreichung einer Abstandsänderung in definierten Schritten an dem einen Ende des Verbindungselements (12) zwei Bohrungen (13) im Abstand von 10 mm und an dem anderen Ende des Verbindungselements (12) eine Vielzahl von Bohrungen (13') im Abstand von 20 mm eingebracht ist.

4. Zielgerät nach einem der vorangegangenen Ansprüche,
**dadurch gekennzeichnet,**
**daß** die Führungsbohrungen (10) in den Schenkeln der U-förmigen Teile der proximalen bzw. distalen Zielvorrichtung (4, 5) zur Berücksichtigung der ventralen bzw. dorsalen Lage der Marknagelrinnen (11) in zwei Ebenen angeordnet sind.

## Claims

1. Aiming device for the X-ray-free proximal and distal locking of intramedullary nails, in which the proximal (5) and the distal aiming device (4) each consist of a U-shaped part, in the legs of which guide bores (10) are made in the widened end regions, the intramedullary nail (6) is firmly connected to the proximal aiming device (5) via a web and the proximal aiming device is connected to the distal aiming device (4) via a pivotable and adjustable spacing fixture (2), and the distance of the intramedullary nail end from the distal aiming device is set by a spacing element (1) such that the centre axes of the guide bores (10) in the aiming devices are in alignment with the centre axes of grooves (11) or bores, which communicate with the guide bores (10), in the intramedullary nail (6),
**characterised in**
**that** the spacing fixture (2) consists of two parts (8, 9) which can slide telescopically in one another such that it can vary in length, that the leg of the U-shaped proximal aiming device (5) is connected to the leg of the U-shaped distal aiming device (4) in the plane of the centre axes of the guide bores (10) or the intramedullary nail grooves (11) by a rigid connection element (12) which is in each case rotatably mounted at the leg of the U-shaped proximal and distal aiming device (4, 5), respectively, and the spacing fixture (2) is disposed at the proximal aiming device (5) via a hinged joint (3) such that it can pivot perpendicularly to the plane of the centre axes of the guide bores (10) or intramedullary nail groves (11).

2. Aiming device according to Claim 1,
**characterised in**
**that** the connection element (12) is a flat steel strip in which bores (13, 13') are made in accordance with the spacing of the guide bores (10) in the proximal and distal aiming device (4, 5), respectively, and a tissue protection sleeve (14) which, as is generally known, is used during operations is passed through the bores (13, 13') and the guide bores (10).

3. Aiming device according to Claim 2,
**characterised in**
**that**, in order to effect a change in spacing in defined steps, two bores (13) are made in one end of the connection element (12) at a spacing of 10 mm, and a plurality of bores (13') are made in the other end of the connection element (12) at a spacing of 20 mm.

4. Aiming device according to any one of the preceding Claims,
**characterised in**
**that** the guide bores (10) in the legs of the U-shaped parts of the proximal and distal aiming device (4, 5), respectively, are disposed in two planes in order to allow for the ventral or dorsal position of the intramedullary grooves (11).

## Revendications

1. Appareil de visée pour le verrouillage proximal et distal sans rayons X de clous intramédullaires, dans lequel le dispositif de visée proximal (5) et le dispositif de visée distal (4) sont constitués, à chaque fois, d'une pièce en forme de U, dans les branches de laquelle, dans les zones d'extrémité élargies, sont prévus des perçages de guidage (10), et dans lequel le clou intramédullaire (6) est relié rigidement au dispositif de visée proximal (5) via une barrette et le dispositif de visée proximal est relié au dispositif de visée distal (4) via un support d'écartement (2) réglable et pouvant pivoter, et dans lequel la distance de l'extrémité du clou intramédullaire du dispositif de visée distal est réglée par un élément d'écartement (1) de sorte que les axes centraux des perçages de guidage (10) dans les dispositifs de visée sont alignés avec les axes centraux de perçages ou rainures (11) communiquant avec les perçages de guidage (10) dans le clou intramédullaire (6),
**caractérisé en ce que** le support d'écartement (2) est constitué, de façon longitudinalement modifiable, de deux parties (8, 9) pouvant coulisser l'une dans l'autre de façon télescopique, **en ce que** la branche du dispositif de visée proximal en forme de U (5) est reliée à la branche du dispositif de visée distal en forme de U (4) dans le plan des axes centraux des perçages de guidage (10) ou des rainures (11) du clou intramédullaire par un élément de liaison rigide (12) qui est monté, à chaque fois, de façon rotative sur la branche du dispositif de visée proximal ou distal en forme de U (4, 5), et **en ce que** le support d'écartement (2) est agencé en pouvant pivoter sur le dispositif de visée proximal (5) via une articulation (3) orthogonalement par rapport au plan des axes centraux des perçages de guidage (10) ou des rainures (11) du clou médullaire.

2. Appareil de visée selon la revendication 1,
**caractérisé en ce que** l'élément de liaison (12) est une bande d'acier plat, dans laquelle, de façon correspondant à l'écartement des perçages de guidage (10) dans le dispositif de visée proximal ou distal (4, 5), des perçages (13, 13') sont prévus, et un manchon de protection en tissu (14) utilisé de façon connue lors de l'opération est enfiché à travers les perçages (13, 13') et les perçages de guidage (10).

3. Appareil de visée selon la revendication 2,
**caractérisé en ce que**, pour obtenir une modification d'écartement à des pas définis, il est prévu, sur une extrémité de l'élément de liaison (12), deux perçages (13) à la distance de 10 mm et, sur l'autre extrémité de l'élément de liaison (12), une pluralité de perçages (13') à la distance de 20 mm.

4. Appareil de visée selon une des revendications précédentes,
**caractérisé en ce que** les perçages de guidage (10) sont agencés dans les branches des pièces en forme de U du dispositif de visée proximal ou distal (4, 5) pour tenir compte de la position ventrale ou dorsale des rainures (11) du clou intramédullaire dans deux plans.
